# EUROPEAN PATENT SPECIFICATION

(11) **EP 2 598 140 B1**
(45) Date of publication and mention of the grant of the patent: **14.01.2015**
(21) Application number: 11744157.6
(22) Date of filing: 29.07.2011
(51) Int. Cl.: A61K 31/381, A61K 31/343, A61K 9/00, A61K 8/42, A61K 8/49, A61K 31/167, A61K 31/4015, A61K 31/421, A61K 31/519, A61P 17/02, A61Q 19/08

(54) **PROSTANOID EP4 AGONISTS FOR USE IN TREATING A SKIN BLEMISH**
PROSTANOID EP4-AGONISTEN ZUR VERWENDUNG BEI DER BEHANDLUNG ZUR HAUT
AGONISTES PROSTANOÏDE EP4 POUR UTILISATION DANS LA REPARATION DE LA PEAU

(30) Priority: 02.12.2010 US 419115 P; 30.07.2010 US 369232 P
(43) Date of publication of application: 05.06.2013
(73) Proprietor: ALLERGAN, INC., Irvine, CA 92612 (US)
(72) Inventor: JIANG, Guang L., Irvine California 92620 (US); BURK, Robert M., Laguna Beach California 92651 (US); IM, Wha Bin, Irvine California 92612 (US); BEDDINGFIELD, Frederick C., Pacific Palisades California 90272 (US); WHEELER, Larry A., Irvine California 92612 (US); WHITCUP, Scott M., Laguna Hills California 92653 (US)
(74) Representative: Hoffmann Eitle
(86) International application number: PCT/US2011/045833
(87) International publication number: WO 2012/016109

(56) References cited:
- EP-A1- 1 114 816
- WO-A1-2007/115001
- WO-A1-2008/071736
- WO-A1-2008/076703
- WO-A2-2009/150118
- CHUN KYUNG-SOO ET AL: "Cyclooxygenase-2 inhibits UVB-induced apoptosis in mouse skin by activating the prostaglandin E-2 receptors, EP2 and EP4", CANCER RESEARCH, vol. 67, no. 5, March 2007 (2007-03), pages 2015-2021, XP002662646, ISSN: 0008-5472
- SAKAI Y ET AL: "Prostaglandin E2 regulates the expression of basic fibroblast growth factor messenger RNA in normal Human Fibroblasts", KOBE JOURNAL OF MEDICAL SCIENCES, KOBE UNIVERSITY SCHOOL OF MEDICINE, KOBE, JP, vol. 47, no. 35-45, 1 February 2001 (2001-02-01), pages 35-45, XP002974917, ISSN: 0023-2513

## Description

### FIELD OF THE INVENTION

The invention relates generally to compositions for wound healing, and particularly to the use of EP4 agonists for treatment in wound healing, scar reduction, and skin repair.

### BACKGROUND OF THE INVENTION

Prostanoid EP4 receptor is a G protein-coupled receptor that mediates the actions of prostaglandin E2 (PGE2) and is characterized by the longest intracellular C terminus loop when compared to other prostanoid receptors. Mainly, EP4 receptors couple to Gs and mediate elevations in cAMP concentration, although they do participate in other pathways as well. There are some redundancies in function between EP2 and EP4 receptors. For example, both receptors induce PGE2-mediated RANKL through cAMP. However, EP2 is involved in cumulus expansion in ovulation and fertilization, whereas EP4 regulates closure of the ductus arteriosus. Expression of EP4 receptors is controlled by various physiological and pathophysiological processes as these receptors participate in ovulation and fertilization, induce bone formation, protect against inflammatory bowel disease, facilitate Langerhans cell migration and maturation and mediate joint inflammation in a model of collagen-induced arthritis, among others.

Skin blemishes such as flesh wounds, scars and wrinkles can occur on any area of the body. Scarring may occur in all parts of adult body, following local or systemic traumas such as mechanical injury, surgery, burn, radiation and poisoning, and represents a failure of homeostatic processes to restore normal structure at the wound sites. Wrinkles occur for a variety of reasons and are a common sign of aging. Both scars and signs of aging can typically considered undesirable.

WO 2007/115001 A1 describes a compound comprising a prodrug of a prostaglandin EP4 agonist, wherein said prodrug is an ester, ether, or amide of an amino acid. Maintenance of the colonic mucosal barrier by a method comprising administering a therapeutically effective amount of a prostaglandin EP4 agonist to a colon of a mammal is also disclosed. Dosage forms, medicaments, and compositions, related thereto are also disclosed.

Sakai et al. (Kobe J. Med. Sci., 2001, v. 47, pp. 35-45) report that PGE2 enhances the expression of bFGF in normal human fibroblasts, and that a calcium ionophore (A23187) and an adenylate cyclase activator (forskolin) also enhace the expression of bFGF mRNA.

Accordingly, an agent that safely and effectively treats or prevents such skin blemishes is highly desirable.

### SUMMARY OF THE INVENTION

The disclosure provides compositions for wound healing and scar reduction. The compositions of the invention include at least one EP4 agonist set forth herein. Wounds and or scars that can be treated by the compositions of the invention can arise from events such as surgery, trauma, disease, mechanical injury, burn, radiation, poisoning, and the like.

In one embodiment of the invention, there are provided compositions for use in methods for treating skin blemishes. Such methods can be performed, for example, by administering to a subject in need thereof a therapeutically effective amount of at least one EP4 agonist, thereby treating the skin blemish.

In one embodiment, a composition is provided for healing a wound that includes administering to a subject in need thereof a composition comprising a therapeutically effective amount of a compound having a structure: 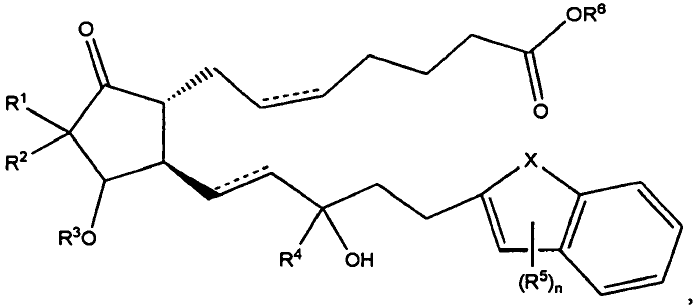
wherein each dashed line represents the presence or absence of a double bond;
R¹, R², R³ and R⁴ are each independently selected from H and C₁-C₆ linear alkyl;
R⁵ is halogen, C₁-C₆ alkyl, or C₁-C₆ alkenyl; R⁶ is H, C₁-C₆ alkyl, C₁-C₆ alkenyl, a salt thereof, or an amine thereof; n is 0-7; and X is S or O.

In another embodiment, a composition is provided for treating a flesh wound that comprises administering a composition comprising a therapeutically effective amount of a compound having a structure: 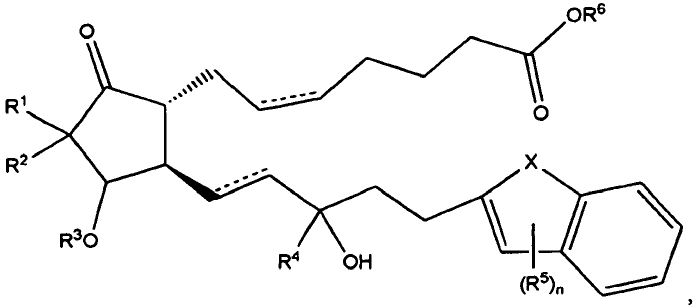
wherein each dashed line represents the presence or absence of a double bond;
R¹, R², R³ and R⁴ are each independently selected from H and C₁-C₆ linear alkyl;
R⁵ is halogen, C₁-C₆ alkyl, or C₁-C₆ alkenyl; R⁶ is H, C₁-C₆ alkyl, C₁-C₆ alkenyl, a salt thereof, or an amine thereof; n is 0-7; and X is S or O,
wherein the wound heals more normally than without administration of the composition.

In yet another embodiment, a method of reducing the appearance of a wrinkle comprising administering to said wrinkle a composition comprising a therapeutically effective amount of a compound having a structure: 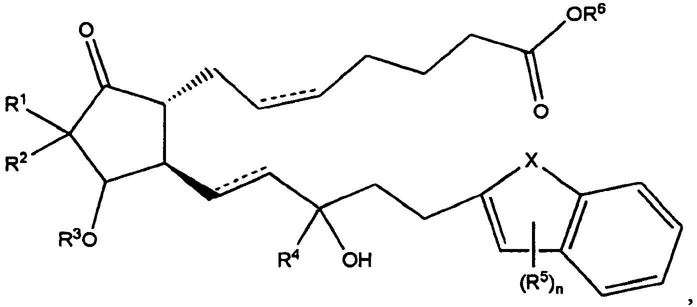
wherein each dashed line represents the presence or absence of a double bond;
R¹, R², R³ and R⁴ are each independently selected from H and C₁-C₆ linear alkyl;
R⁵ is halogen, C₁-C₆ alkyl, or C₁-C₆ alkenyl; R⁶ is H, C₁-C₆ alkyl, C₁-C₆ alkenyl, a salt thereof, or an amine thereof; n is 0-7; and X is S or O,
wherein the appearance of the wrinkle is diminished.

### DETAILED DESCRIPTION OF THE INVENTION

Disclosed herein are compositions for wound healing and scar reduction. In one embodiment the compositions described herein comprise compounds having a general structure: 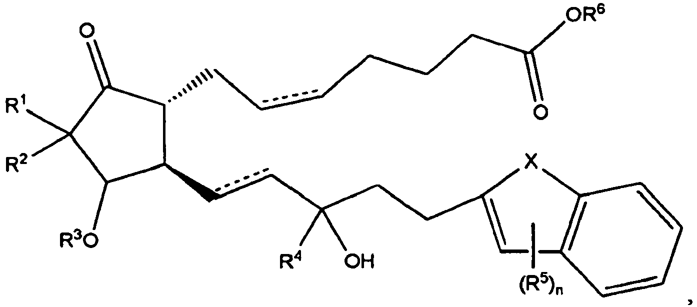
wherein each dashed line represents the presence or absence of a double bond;
R¹, R², R³ and R⁴ are each independently selected from H and C₁-C₆ linear alkyl;
R⁵ is halogen, C₁-C₆ alkyl, or C₁-C₆ alkenyl; R⁶ is H, C₁-C₆ alkyl, C₁-C₆ alkenyl, a salt thereof, or an amine thereof; n is 0-7; and X is S or O.

In certain embodiments, R⁴ is H, R³ is H, and X is S.

In another embodiment, R¹ and R² are CH₃.

In a further embodiment, R⁵ is Cl.

In yet another embodiment, the compound is:

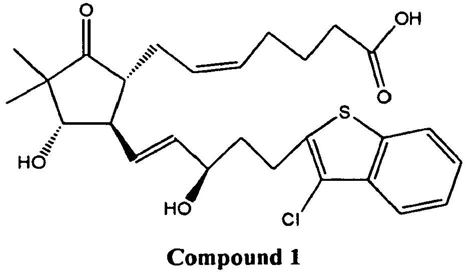

Methods of preparing the disclosed compounds and additional compounds suitable for use in the methods disclosed herein, can be found in, e.g., Donde, et el., 10,10-Dialkyl Prostanoic Acid Derivatives as Agents for Lowering Intraocular Pressure, U.S. Patent 6,875,787; Donde, et el., 10,10-Dialkyl Prostanoic Acid Derivatives as Agents for Lowering Intraocular Pressure, U.S. Patent Publication 2004/0235958; Donde, et al., Treatment of Inflammatory Bowel Disease, U.S. Patent Publication 2005/0164992.

As used herein, the term "skin blemish" includes a flesh wound, scar, or wrinkle on any region of the skin of a body.

A "flesh wound" can be any area in which the structural integrity of the exterior surface of the skin is compromised. A flesh wound can be due to incision, laceration, abrasion, thermal burn, chemical burn, radiation or puncture of the skin. The wound can be superficial or extend to the deeper layers of the dermis, subcutaneous, deep fascia, muscle, bone or other internal organs.

A "scar" is an area of fibrous tissue (fibrosis) that replaces normal skin (or other tissue) after injury or disease. Scar types include hypertrophic scars, recessed scars, and stretch marks. Hypertrophic scars occur when the body overproduces collagen, which causes the scar to be raised above the surrounding skin. An example of a hypertrophic scar is a keloid scar. Atrophic, or recessed scars, have a sunken appearance and result when underlying support structure in the skin is lost. Stretch marks (striae) occur when skin is stretched rapidly (i.e., due to significant weight gain or growth spurt), or when skin is put under tension during the healing process, typically near a joint. As used herein, the term "scar" encompasses any type of scar in the skin due to any cause.

As used herein, the term "wrinkle" is a fold, ridge, crease, furrow, pit, crater, or sunken area in the skin that can be caused by habitual facial expressions, loss of collagen and/or elasticity due to aging, sun damage, smoking, poor hydration, and various other factors. A wrinkle can range from a deep crease to a fine line. Wrinkles occurring on any part of a body, in particular, wrinkles on head or neck of a subject are contemplated herein. Wrinkles that can be treated in accordance with the disclosure include, but are not limited to, a brow furrow, crows feet, nasolabial fold, one or more lines under the eyes or between the eye brows, and combinations thereof.

As used herein, "treatment" means to alleviate (or to eliminate) one or more features of a skin blemish either temporarily or permanently. When the compositions are administered to treat a wound, the compositions promote normal healing compared to a wound without the administration. That is, the size (length, depth, height and/or width), character, color and/or texture of the treated wound more closely resemble normal, non-wounded tissue. In this regard, treatment of a wound with the disclosed compositions can prevent, minimize or improve the appearance of a scar formation resulting from healing of the wound. Further, when the disclosed compositions are administered to treat a wrinkle, the wrinkle is treated if the appearance or prominence of the wrinkle is visibly or clinically diminished. That is the length and/or depth is decreased compared to the wrinkle prior to treatment. Alternatively, treatment can comprise prevention of a wrinkle. In this regard, the disclosed compositions can be applied to a region of the skin that typically develops a wrinkle, such as a forehead, lips, eyelids, nasolabial fold, skin under an eye, or between the eye brows in order to prevent the development of a wrinkle.

The disclosed compositions can be administered to prevent scar formation not associated with a wound, such as a stretch mark, or scars resulting from acne, chicken pox, measles or other disease states. In certain embodiments, the disclosed compositions are administered to the area of skin expansion in order to prevent formation of such scars. In these embodiments, the composition can be administered to any region of a face, abdomen, breasts, arms, legs, buttocks, back, or any other area where the skin is susceptible to developing a scar.

The compositions can be administered prior to, concurrently with, and/or after the development of the skin blemish. For instance, the disclosed compositions can be administered prior to an incision, during a surgical procedure, and/or any time post-operatively, and then additionally administered after the procedure as the healing process occurs. In another example, the compositions can be administered during pregnancy to prevent stretch marks. Alternately, the compositions can be administered after the development of a blemish.

The compositions may be administered between 1 and 7 days a week, for a period of time necessary to achieve the desired results, which may be several days to several months. The compositions can be administered once or several times (2, 3, 4, or more times) a day depending on the desired effect. In certain embodiments, the compositions can be administered every 1, 2, 3, 4, 5, 6, or 7 days. In another embodiment, the compositions can be administered one or more times every 1, 2, 3, or 4 weeks. The administration can be on a monthly or bi-monthly basis. Further, the compositions can be administered for 1, 2, 3, 6, 9, or 12 months or more. In certain embodiments, the compositions can be administered on an ongoing basis to maintain a desired result.

The disclosed compounds can be administered as part of a composition. As used herein, "formulation" and "composition" may be used interchangeably and refer to a combination of elements that is presented together for a given purpose. Such terms are well known to those of ordinary skill in the art.

As used herein, "carrier," "inert carrier," and "acceptable carrier" may be used interchangeably and refer to a carrier which may be combined with the presently disclosed compounds in order to provide a desired composition. Those of ordinary skill in the art will recognize a number of carriers that are well known for making specific pharmaceutical and/or cosmetic compositions. Desirably, the carrier is suitable for application to keratinous surfaces or other areas of the body. Upon application, acceptable carriers are substantially free of adverse reactions with skin and other keratinous surfaces. For example, the carriers may take the form of fatty or non-fatty creams, milky suspensions or emulsion-in-oil or oil-in-water types, lotions, gels or jellies, colloidal or non-colloidal aqueous or oily solutions, pastes, aerosols, soluble tablets or sticks. In accordance with one embodiment, the composition includes a dermatologically compatible vehicle or carrier. The vehicle which may be employed for preparing compositions may comprise, for example, aqueous solutions such as e.g., physiological salines, oil solutions or ointments. The vehicle furthermore may contain dermatologically compatible preservatives such as e.g., benzalkonium chloride, surfactants like e.g., polysorbate 80, liposomes or polymers, for example, methyl cellulose, polyvinyl alcohol, polyvinyl pyrrolidone and hyaluronic acid; these may be used for increasing the viscosity.

Examples of additional agents which can be included in the present compositions are anti-itch, anti-cellulite, anti-scarring, and anti-inflammatory agents, anesthetics, anti-irritants, vasoconstrictors, vasodilators, as well as agents to prevent/stop bleeding, and improve/remove pigmentation, moisturizers, desquamating agents, tensioning agents, anti-acne agents. Anti-itch agents can include methyl sulphonyl methane, sodium bicarbonate, calamine, allantoin, kaolin, peppermint, tea tree oil and combinations thereof. Anti-cellulite agents can include forskolin, xanthine compounds such as, but not limited to, caffeine, theophylline, theobromine, and aminophylline, and combinations thereof. Anesthetic agents can include lidocaine, benzocaine, butamben, dibucaine, oxybuprocaine, pramoxine, proparacaine, proxymetacaine, tetracaine, and combinations thereof. Anti-scarring agents can include IFN-gamma, fluorouracil, poly(lactic-co-glycolic acid), methylated polyethylene glycol, polylactic acid, polyethylene glycol and combinations thereof. Anti-inflammatory agents can include dexamethasone, prednisolone, corticosterone, budesonide, estrogen, sulfasalazine, mesalamine and derivatives and combinations thereof. Additionally, active agents such as epinephrine, thymidine, cytidine, uridine, antiypyrin, aminocaproic acid, tranexamic acid, eucalyptol, allantoin, glycerin, and sodium selenite, can be included. Formulations can further comprise degradation inhibitors. Degradation inhibitors, include but are not limited to, glycosaminoglycans (e.g., heparin, heparin sulfate, dermatan sulfate, chrondroitin sulfate, o-sulfated HA, Inamarin, and amygdalin), antioxidants (e.g. ascorbic acid, melatonin, vitamin C, vitamin E), proteins (e.g., serum hyaluronidase inhibitor), and fatty acids (e.g. saturated C₁₀ to C₂₂ fatty acids). In certain embodiments, additional active agent is an antioxidant. In certain embodiments, the antioxidant comprises a vitamin C and/or a vitamin E such as d-alpha-tocopheryl polyethylene glycol 1000 succinate (TPGS).

The disclosed compositions are well suited for topical, subcutaneous, intradermal, subdermal, and trandermal administration. Topical administration relates to the use of a composition applied to the surface of the skin at the site of a skin blemish for exertion of local action. Accordingly, such topical compositions include those pharmaceutical or cosmetic forms in which the composition is applied externally by direct contact with the skin surface to be treated, such as the face, neck, arms, legs, and/or torso. Conventional pharmaceutical or cosmetic forms for this purpose include ointments, liniments, creams, shampoos, lotions, pastes, jellies, sprays, aerosols, and the like, and may further be applied directly or in patches or impregnated dressings depending on blemish and skin region to be treated. The term "ointment" embraces formulations (including creams) having oleaginous, water-soluble and emulsion-type bases, e.g., petrolatum, lanolin, polyethylene glycols, as well as mixtures of these.

The compositions are appropriate for mesotherapy applications as well. Mesotherapy is a non-surgical cosmetic treatment technique involving intra-epidermal, intra-dermal, and/or subcutaneous injection of a composition. The compositions are administered in the form of small multiple droplets into the epidermis, dermo-epidermal junction, and/or the dermis.

In accordance with the disclosure, a pharmaceutical or cosmetic composition can optionally include one or more agents such as, without limitation, emulsifying agents, wetting agents, sweetening or flavoring agents, tonicity adjusters, preservatives, buffers antioxidants and flavonoids. Tonicity adjustors useful in a pharmaceutical composition of the present disclosure include, but are not limited to, salts such as sodium acetate, sodium chloride, potassium chloride, mannitol or glycerin and other pharmaceutically acceptable tonicity adjusters. Preservatives useful in the pharmaceutical compositions described herein include, without limitation, benzalkonium chloride, chlorobutanol, thimerosal, phenyl mercuric acetate, and phenyl mercuric nitrate. Various buffers and means for adjusting pH can be used to prepare a pharmaceutical composition, including but not limited to, acetate buffers, citrate buffers, phosphate buffers and borate buffers. Similarly, antioxidants useful in pharmaceutical compositions are well known in the art and include for example, sodium metabisulfite, sodium thiosulfate, acetylcysteine, butylated hydroxyanisole and butylated hydroxytoluene. Flavonoids are compounds found in plants that are well known to have diverse beneficial biochemical and antioxidant effects. Subcategories of flavonoids include: flavones, flavonols, flavanonse and flavanonols. Examples of flavonoids include: luteolin, apigenin, tangeritin, quercetin, kaempferol, myricetin, fisetin, isorhamnetin, pachypodol, rhamnazin, hesperetin, naringenin, eriodictyol, homoeriodictyol, taxifolin, dihydroquercetin, dihydrokaempferol, tannic acid, tannis, condensed tannis, and hydrolysable tannis. It is understood that these and other substances known in the art can be included in a pharmaceutical or cosmetic composition disclosed herein.

As used herein, the term "therapeutically effective amount" means the amount of the pharmaceutical or cosmetic composition that will elicit the biological, medical, or cosmetic response of a subject in need thereof that is being sought by the researcher, veterinarian, medical doctor or other clinician. In some embodiments, the subject in need thereof is a mammal. In certain embodiments, the mammal is human. Effective amounts of the compound may be determined by one of ordinary skill in the art but will vary depending on the compound employed, frequency of application and desired result, and will generally range from about 0.0000001% to about 50%, by weight, of the composition, preferably from about 0.001% to about 50%, by weight, of total composition, more preferably from about 0.001% to about 30%, by weight of the composition. In certain embodiments, the compound is about 0.004% by weight of the composition.

The compounds described herein may be administered at least in the minimum dose necessary to achieve the desired therapeutic effect. Generally, such doses will be in the range of about 1 mg/day to about 1000 mg/day; more preferably in the range of about 10 mg/day to about 500 mg/day. In another example embodiment, the compound or compounds may be present in a composition or formulation in a range of about 0.0001 mg/kg/day to about 100 mg/kg/day or about 0.01mg/kg/day to about 100 mg/kg/day. However, the actual amount of the compound to be administered in any given case will be determined by a physician taking into account the relevant circumstances, such as the age and weight of a patient, patient's general physical condition, severity of the skin blemish, and route of administration. In some instances, dosing is evaluated on a case-by-case basis.

Additionally, compositions may be designed to delay release of the compound over a given period of time, or to carefully control the amount of compound released at a given time during the course of treatment.

The pH of the disclosed compositions can be about 3 to about 8.0, or about 6.5 to about 7.5. In certain embodiments, the pH of the formulation is about 7.0 to about 7.4 or about 7.1 to about 7.3.

### EXAMPLE 1

### The Effect of Compound 1 on Wound Healing

Incisional Skin Wound Model and Assessment. Sprague-Dawley rats at 180-200 gram were anesthetized with isoflourane. After shaving, a 2-cm long incision was made, reaching the deep fascia on the back skin of rats under sterile conditions. The wounds were immediately closed with 4-0 sutures. A 14 day pilot study was carried out. The animals were topically treated with vehicle or Compound 1 at 0.004% twice daily. The vehicle contained ethanol 30%, propylene glycol 12%, dipropylene glycol 5%, benzyl alcohol 5%, glycerol 3% and normal saline 45%. The wound was photographed daily; biopsy was performed at 2, 3, 7 and 14 days post-surgery for histopathology and molecular biology analysis.

A similar skin wound study was also performed comparing the effects of Compound 1 and TGF-β3. In this study, intradermal injections of Compound 1 at 0.004%, TGF-β3 at 100 ng/200 µl or vehicle were given right before closing the wounds. Afterward, TGF-β3 was injected two more times, on day 1 and 2, and Compound 1 and vehicle were topically applied twice a day for the duration of the study. The vehicle was PBS with 0.1% BSA and 4 mM HCl in a total volume of 200 µl for injection. Skin wounds were imaged on day 3, 7, 14, 35 and 70.

The wound tissue was biopsied for histopathology on day 3, 14 and 70. To observe the skin wound, paraffin-embedded wound sections were made. Regular H&E staining was carried out in comparison with Masson trichrome and/or Picosirus red to visualize the collagen fibers. To monitor myofibroblasts in skin wound, the sections were immunohistochemically stained to identify alpha-smooth muscle actin. To assess wound appearance, all the scar photos were mixed together by the end of each study. The scar severity was scored on a scale of 0 to 10, with 0 being invisible, 1 the minimal and 10 the worst. Each scar was divided into 4 regions, separated by suture sites; each quarter was scored independently; the mean of the 4 part scores was recorded as the gross score of each wound.

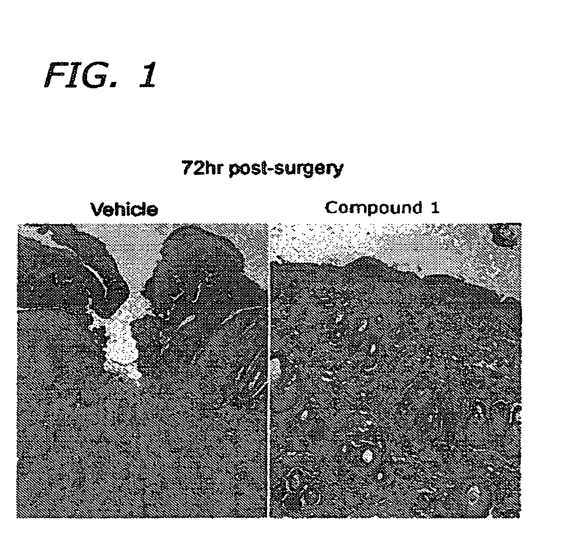

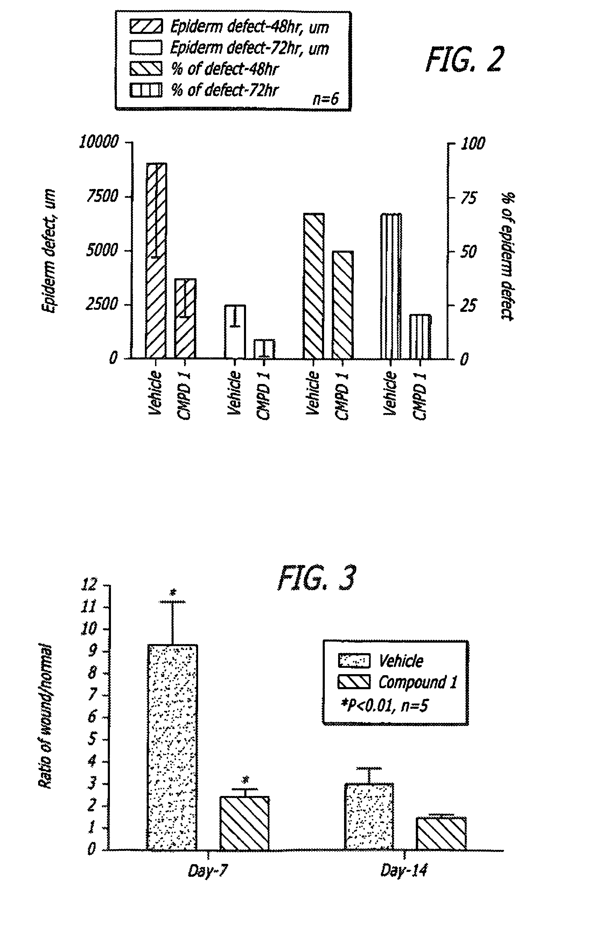

On day 3, 80% of skin wound samples treated with Compound 1 showed closed epidermis filled with keratinocytes, while only 33% of vehicle treated wounds had closed epidermis (Figures 1 and 2). The overall size of epidermal defects was two times larger for vehicle-treated wounds as compared with that of Compound 1 treated wounds (Figures 1 and 2). This demonstrates a beneficial effect of the Compound 1 treatment on the healing of the epidermal layer.

On 7 days post-skin incision, the epidermal layer of Compound 1 treated skin not only had a thickness close to the nearby normal epidermis, but also had epidermal wrinkle resembling normal elastic skin structure. In contrast, the vehicle-treated skin had epidermal hyperplasia with a thickness of 3 times more than the Compound 1 treated epidermis (Figure 3).

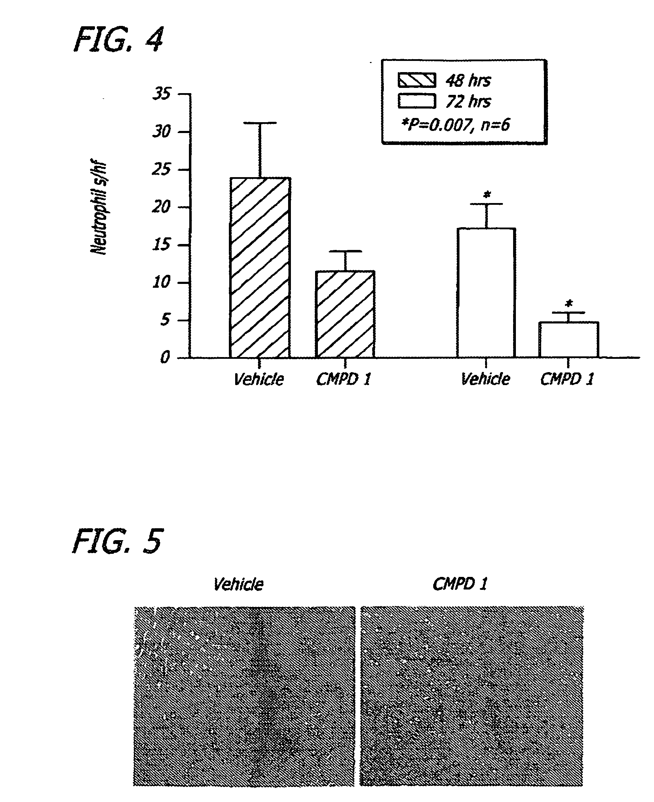

Neutrophils are recruited to injury sites as the first innate immune response. Their lysis and release of chemokines attract other inflammation cells and amplify inflammatory processes. Neutrophil infiltration was monitored on sectioning tissue on days 2 and 3. Compound 1 significantly reduced polymorphonuclear cell infiltration at wound sites (Figure 4).

Myofibroblasts were identified by immunohistochemical staining of alpha-smooth muscle actin (α-SMA) on sections from day 2 to day 14 post-surgery. Both staining and assessment were conducted by personnels blinded to the treatments. Strong α-SMA signals were localized at the cytoplasm of large cells, and such α-SMA-positive cells were mainly distributed along the granulation tissue at the dermis layer at wound sites. Abundant myofibroblasts were observed on day 3 samples, which indicated their proliferation during adult scar wound healing. Compound 1 treatment reduced the number of myofibroblasts (25.8±7.45/3 sections) as compared to vehicle control (38±6.15/3 sections).

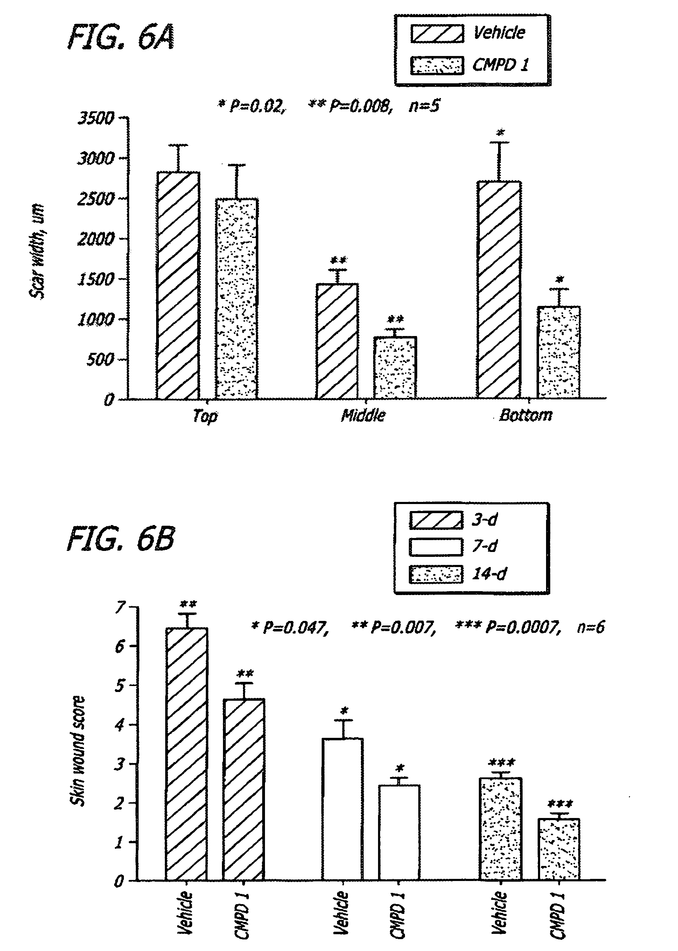

Biopsy samples of skin wound tissues were analyzed at 7 and 14 days post-surgery. Tissue samples about 1 mm wide were taken from both sides of the wound. Sections from day 14 were stained for collagen fibers by Masson Trichrome. The scar sites contained fine, short, lightly stained collagen fibers, positioning somewhat parallel to the epidermis, but generally in unstructured fashion. In normal dermis, the collagen fibers were thick, long, deeply stained, and clearly organized in a basket-weave mode, which appears to be central to the elasticity and tensile of normal skin. The width of the abnormal fiber belt was measured at the surface, the middle and the bottom of scars. Compound 1 treatment significantly reduced the width in the middle and bottom parts of scars, but displayed only a tendency to decrease scar width at the superficial region (Figures 5 and 6A and B). Grossly, Compound 1 treated animals had smaller and softer skin scar, and significantly slimmer appearances than vehicle-treated animals (Figures 5 and 6A and B).

Since TGF-β3 is a leading treatment for wounds, reportedly reducing skin scar in both animals and human, the effect of Compound 1 and TGF-β3 were compared. Here, the focus was on three temporal phases of wound healing and scar formation: inflammation on day 3, overall wound healing on day 14, and scar remodeling on day 70. Neutrophil infiltration, a hall mark of inflammation, was easily detectable 3 days post-surgery. The number of neutrophils was counted in three sections of H&E stained tissues; they were 60.6±30, 53.8±17 or 31.4±8 for vehicle, TGF-β3 or Compound 1 treated groups, respectively. The trend of suppressed neutrophil infiltration by Compound 1 was apparent, albeit not statistically significant due to small samples (n=5), and is consistent with our previous observation.

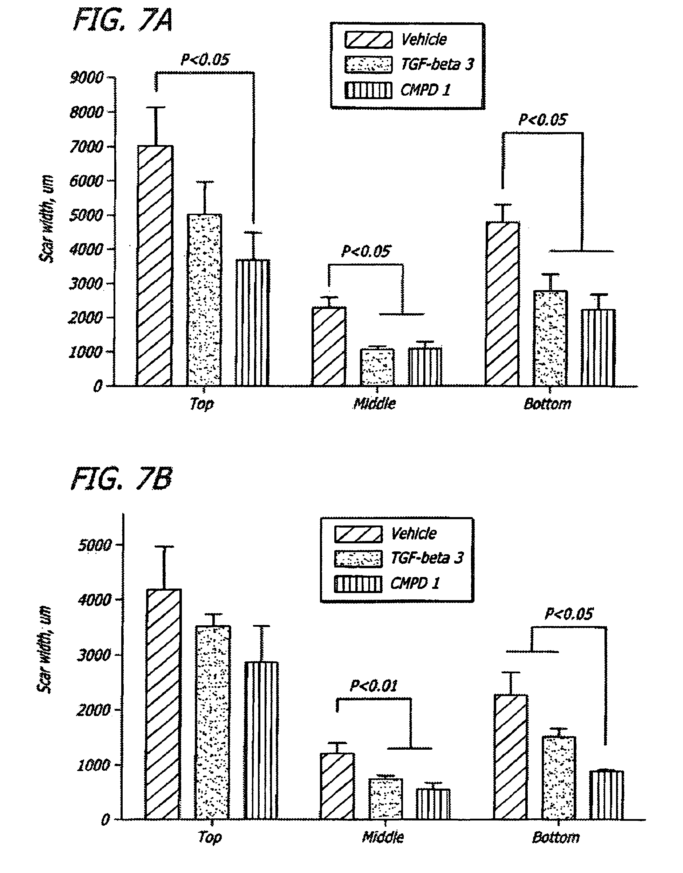

At day 14, wounded skin tissue was processed for both Picrosirius red and Masson trichrome collagen staining. For Picrosirius-stained tissues under polarized light, type I collagen fibril appears in yellow color and type III collagen in green. Vehicle-treated wounds showed some green, fine fibrils in gaps, but not yellow, large fibril bundles. The TGF-(33-treatment also had some green fibers at the bottom of the wounds, but Compound 1 treatment showed large yellow-stained collagen bundles almost crossing over the entire wound sites, with little green-stained type III collagen (Figures 7A and B). Also the gap width in-between the normal fibrils was significantly narrower in both TGF-β3-treated and Compound 1 treated groups than that of vehicle-treated group (p<0.05, Figures 7A and B). This indicated that Compound 1 treatment not only reduced the abnormal structured gap but also diminished immature type III collagen at wound sites.

Different sections of the same wounds were also processed for Masson trichrome collagen staining. Collagen at nearby normal skin was stained as dark-blue, thick bundle oriented in a basket-weave reticular pattern. A distinctive region at the wound site was stained as fine, thin collagen fibers in parallel to epidermis. The demarcation between normal and abnormal region was quite clear. The widths of the abnormal structured dermis regions were significantly smaller in both TGF-β3 and Compound 1 treated groups than that of vehicle treated group (p<0.01-0.05, Figures 7A and B).

Skin wound at a later phase undergoes remodeling. At 70 days post-surgery, wound sites showed different features of collagen staining from those seen 14 days post surgery. On Picrosirius red stained sections, wound gaps in vehicle-treated group were now filled by dense, red, fine fibers in a parallel orientation. Such abnormal regions were largely absent in both TGF-β3 and Compound 1 treated groups. Instead, more abundant yellow, thick bundles of collagen fibers in a basket-weave pattern was observed than the vehicle treated skin.

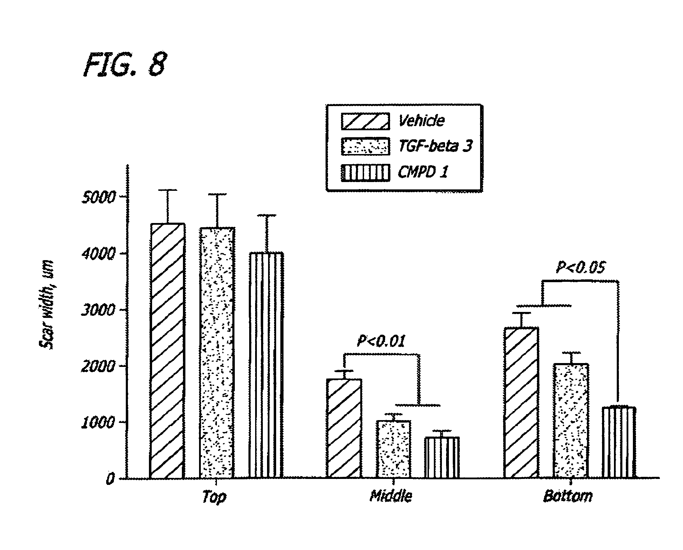

Masson trichrome staining also revealed temporal changes in scar remodeling. On day 70, the scar regions were filled with fine, thin collagen fibers more densely than on day 14. The demarcation between normal and abnormal region became much more distinctive than on day 14. The size of residual scar regions was remarkably smaller in both TGF-β3 and Compound 1 treated groups than that of vehicle treated group. The effect of Compound 1 was more noticeable than TGF-β3 (p<0.01-0.05, Figure 8).

Also the macroscopic surface appearance of wound sites was monitored 70 days post-surgery. In the vehicle treatment, wound sites were replaced with white, shiny, firm, slightly raised scars. The TGF-β3 treatment still showed traces of wounds, although much improved over the vehicle treatment. With the Compound 1 treatment, wound sites were not even detectable, if not for two indication markings on the tissue.

### EXAMPLE 2

### Effect of Compound 1 on Collagen production

The effect of Compound 1 on collagen production was assessed in cultured human fetal and adult skin fibroblasts. Fetal skin fibroblasts were generated from normal skin of 14 weeks gestation fetus, purchased from ATCC (CRL-7129). Adult skin fibroblasts were derived from normal skin of a 61-year old Caucasian female, purchased from ATCC (CRL-7346). Both cells were cultured in DMEM medium supplemented with 10% fetal bovine serum and 1% Streptomycin and Penicillin in incubators at 37°C and 5% CO2. Cells were seeded in 10 cm dishes at 1 x 10⁶ cells/dish. When the cells become 80% confluent, vehicle,or compound 1 was added to culture medium at 0 or 10 nM final concentration, respectively. Compound 1 was first dissolved in DMSO, the final DMSO concentration was 0.1%. Cell lysates were collected at 10, 30, 60, 120 minutes and 24 hours after treatments, respectively. Proteins were quantitated and resolved on 4-10% SDS-PAGE. Then the proteins were transferred to membrane by electrophoresis. The membranes were blocked with mouse-anti-Akt or pAkt, and second antibody against mouse-IgG conjugated with AP (purchased from Signal transduction).

Compound 1 or vehicle with or without Akt inhibitor (5 µM) were added to culture medium for 48 hours. For the dose-response study, the cells were treated for 48 hours at concentration of 0, 3 or 10 nM. Cell lysates were collected and proteins were resolved as above. First antibody was mouse-anti-collagen type I (Millipore). This is a monoclonal IgG1 antibody reacting only with native, non-denatured Collagen I, no cross reactivity with collagen types III, V and VI or connective tissue protein.

Collagen type-1 production was upregulated in fetal and adult skin fibroblasts treated with Compound 1 (see Table 1).

**Table 1**

| | Vehicle | Compound 1 |
|---|---|---|
| Fetal skin fibroblasts | 100% | 200% |
| Adult skin fibroblasts | 100% | 128% |

## Claims

1. A pharmaceutical composition for use in a method of treating a skin blemish, the composition comprising a therapeutically effective amount of a compound having a structure: 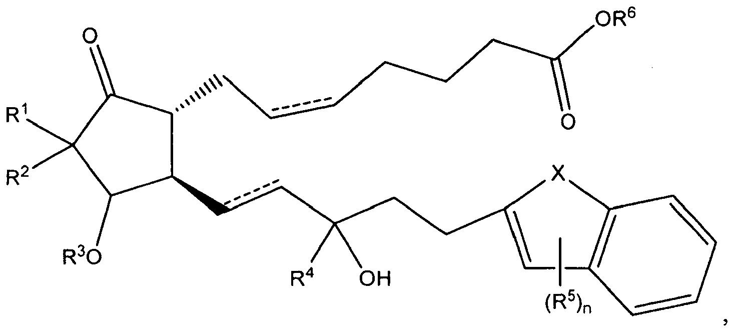
wherein each dashed line represents the presence or absence of a double bond;
R¹, R², R³ and R⁴ are each independently selected from H and C₁-C₆ linear alkyl;
R⁵ is halogen, C₁-C₆ alkyl, or C₁-C₆ alkenyl; R⁶ is H, C₁-C₆ alkyl, C₁-C₆ alkenyl, a salt thereof, or an amine thereof; n is 0-7; and X is S or O.

2. The composition for use according to claim 1, wherein R⁴ is H, R³ is H, and X is S.

3. The composition for use according to claim 1, wherein R¹ and R² are CH₃.

4. The composition for use according to claim 1, wherein R⁵ is Cl.

5. The composition for use according to claim 1, wherein the compound is:

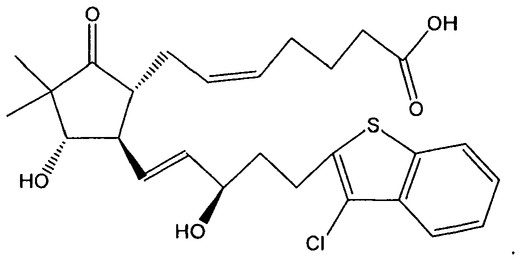

6. The composition for use according to claim 1, wherein the skin blemish is a flesh wound or scar.

7. The composition for use according to claim 1, wherein the composition is administered susubcutaneous, subdermal or transdermal, intradermally or topically.

8. The composition for use according to claim 6, wherein the administration reduces formation of a scar type selected from the group consisting of hypertrophic scar, recessed scar, stretch mark, and a combination thereof.

9. The composition for use according to claim 1, wherein the composition is administered at a time selected from the group consisting of prior to surgical incision, during surgery, post-operatively, and a combination thereof.

10. The composition for use according to claim 1, wherein said administration minimizes or prevents scar formation.

11. The composition for use according to claim 6, wherein a cause of said flesh wound is selected from the group consisting of an incision, a laceration, a thermal burn, a chemical burn, an abrasion, a puncture wound, and a combination thereof.

12. A method of preventing or reducing the appearance of a wrinkle comprising administering to a region of the skin that typically develops a wrinkle or an existing wrinkle a cosmetic composition comprising an effective amount of a compound having a structure: 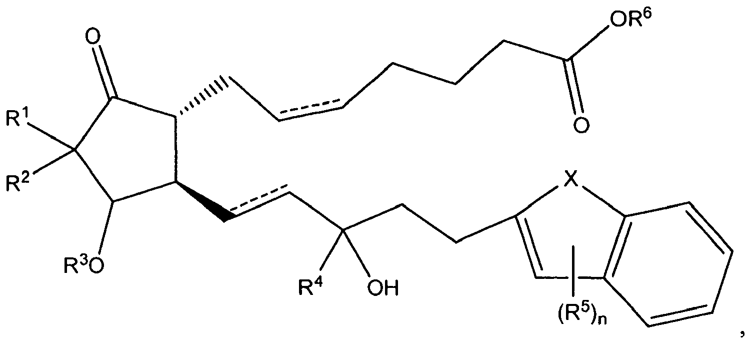
wherein each dashed line represents the presence or absence of a double bond;
R¹, R², R³ and R⁴ are each independently selected from H and C₁-C₆ linear alkyl;
R⁵ is halogen, C₁-C₆ alkyl, or C₁-C₆ alkenyl; R⁶ is H, C₁-C₆ alkyl, C₁-C₆ alkenyl, a salt thereof, or an amine thereof; n is 0-7; and X is S or O.

13. The method of claim 12, wherein the compound is:

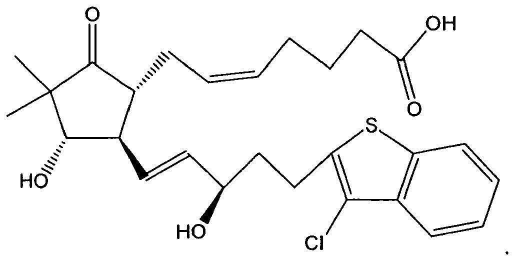

14. The method of claim 12, wherein said composition is administered topically.

15. The method of claim 12, wherein the wrinkle is selected from the group consisting of a brow furrow, crow's feet, nasolabial fold, a line under the eye, a crease between the eye brows, and a combination thereof.

## Patentansprüche

1. Pharmazeutische Zusammensetzung zur Verwendung in einem Verfahren zur Behandlung von Hautfehlern, wobei die Zusammensetzung eine therapeutisch wirksame Menge einer Verbindung mit der Struktur: 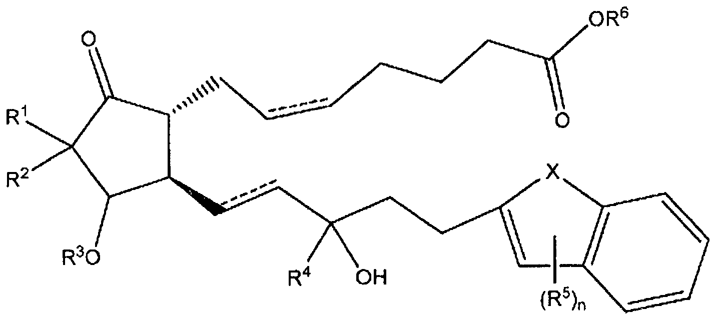 umfasst, worin jede gestrichelte Linie die Gegenwart oder Abwesenheit einer Doppelbindung darstellt; R¹, R², R³ und R⁴ jeweils unabhängig aus H und geradkettigem C₁₋₆-Alkyl ausgewählt sind; R⁵ Halogen, C₁₋₆-Alkyl oder C₁₋₆-Alkenyl ist; R⁶ H, C₁₋₆-Alkyl, C₁₋₆-Alkenyl, ein Salz davon oder ein Amin davon ist;
n 0 bis 7 ist und X S oder O ist.

2. Zusammensetzung zur Verwendung gemäss Anspruch 1, worin R⁴ H ist, R³ H ist und X S ist.

3. Zusammensetzung zur Verwendung gemäss Anspruch 1, worin R¹ und R² CH₃ sind.

4. Zusammensetzung zur Verwendung gemäss Anspruch 1, worin R⁵ Cl ist.

5. Zusammensetzung zur Verwendung gemäss Anspruch 1, worin die Verbindung 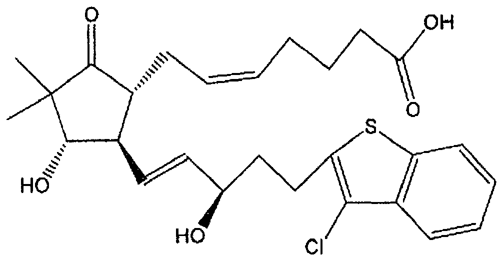 ist.

6. Zusammensetzung zur Verwendung gemäss Anspruch 1, wobei der Hautfehler eine Fleischwunde oder eine Narbe ist.

7. Zusammensetzung zur Verwendung gemäss Anspruch 1, wobei die Zusammensetzung subkutan, subdermal oder transdermal, intradermal oder topisch verabreicht wird.

8. Zusammensetzung zur Verwendung gemäss Anspruch 6, wobei die Verabreichung die Bildung von Narbentypen, ausgewählt aus der Gruppe bestehend aus hypertrophen Narben, vertieften Narben, Dehnungsstreifen und einer Kombination davon, reduziert.

9. Zusammensetzung zur Verwendung gemäss Anspruch 1, wobei die Zusammensetzung zu einem Zeitpunkt, ausgewählt aus der Gruppe bestehend aus vor einer chirurgischen Inzision, während einer Operation, nach einer Operation und einer Kombination davon, verabreicht wird.

10. Zusammensetzung zur Verwendung gemäss Anspruch 1, wobei die Verabreichung die Bildung von Narben minimiert oder verhindert.

11. Zusammensetzung zur Verwendung gemäss Anspruch 6, wobei die Ursache der Fleischwunde aus der Gruppe bestehend aus einer Inzision, einer Lazeration, einer thermischen Verbrennung, einer chemischen Verbrennung, einer Abschürfung, einer Stichwunde und einer Kombination davon ausgewählt ist.

12. Verfahren zur Prävention oder Reduzierung der Erscheinung von Falten, umfassend die Verabreichung einer kosmetischen Zusammensetzung, die eine wirksame Menge einer Verbindung mit der Struktur: 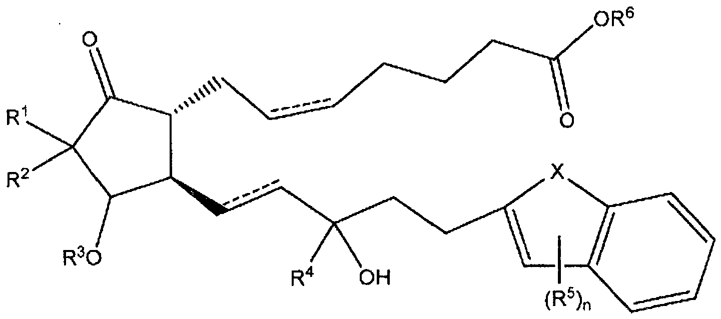 umfasst, worin jede gestrichelte Linie die Gegenwart oder Abwesenheit einer Doppelbindung darstellt; R¹, R², R³ und R⁴ jeweils unabhängig aus H und geradkettigem C₁₋₆-Alkyl ausgewählt sind; R⁵ Halogen, C₁₋₆-Alkyl oder C₁₋₆-Alkenyl ist; R⁶ H, C₁₋₆-Alkyl, C₁₋₆-Alkenyl, ein Salz davon oder ein Amin davon ist;
n 0 bis 7 ist und X S oder O ist, an einen Bereich, der typischerweise Falten entwickelt, oder eine bestehende Falte.

13. Verfahren gemäss Anspruch 12, wobei die Verbindung 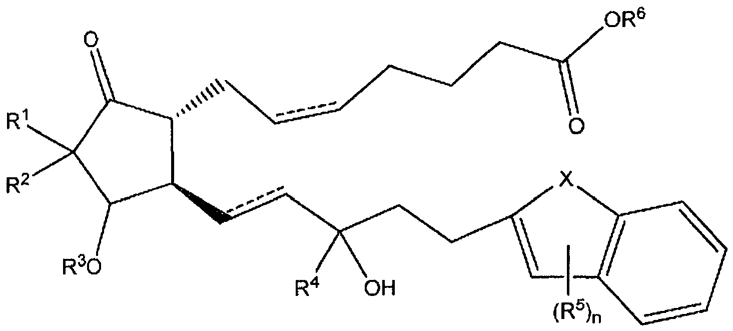 ist.

14. Verfahren gemäss Anspruch 12, wobei die Zusammensetzung topisch verabreicht wird.

15. Verfahren gemäss Anspruch 12, wobei die Falte aus der Gruppe bestehend aus einer Stirnfurche, Krähenfüssen, einer Nasolabialfalte, einer Linie unter dem Auge, einer Falte zwischen den Augenbrauen und einer Kombination davon ausgewählt ist.

## Revendications

1. Composition pharmaceutique pour une utilisation dans un procédé de traitement d'une imperfection de la peau, la composition comprenant une quantité thérapeutiquement efficace d'un composé ayant une structure : 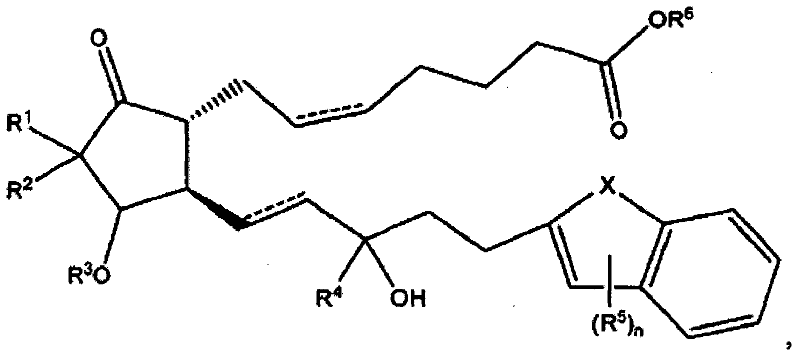 dans laquelle chaque ligne en pointillés représente la présence ou l'absence d'une double liaison ;
R¹, R², R³ et R⁴ sont choisis chacun indépendamment parmi H et un groupe alkyle en C₁ à C₆ linéaire ; R⁵ représente un atome d'halogène, un groupe alkyle en C₁ à C₆, ou alcényle en C₁ à C₆ ; R⁶ représente H, un groupe alkyle en C₁ à C₆, alcényle en C₁ à C₆, l'un de ses sels, ou l'une de ses amines ; n vaut 0 à 7 ; et X représente S ou O.

2. Composition pour une utilisation selon la revendication 1, où R⁴ représente H, R³ représente H, et X représente S.

3. Composition pour une utilisation selon la revendication 1, où R¹ et R² représentent CH₃.

4. Composition pour une utilisation selon la revendication 1, où R⁵ représente Cl.

5. Composition pour une utilisation selon la revendication 1, dans laquelle le composé est :

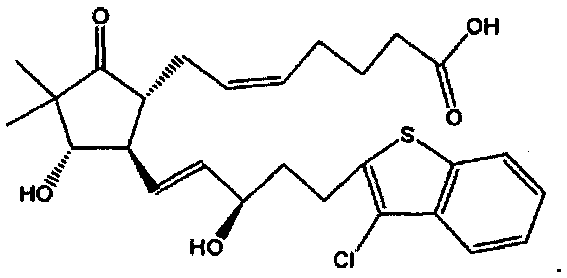

6. Composition pour une utilisation selon la revendication 1, où l'imperfection de la peau est une plaie dans la chair ou une cicatrice.

7. Composition pour une utilisation selon la revendication 1, où la composition est administrée par voie sous-cutanée, sous-dermique ou transdermique, intradermique ou topique.

8. Composition pour une utilisation selon la revendication 6, où l'administration réduit la formation d'un type de cicatrice choisi dans le groupe constitué d'une cicatrice hypertrophique, une cicatrice en creux, une vergeture, et l'une de leurs combinaisons.

9. Composition pour une utilisation selon la revendication 1, où la composition est administrée à un moment choisi dans le groupe constitué d'avant une incision chirurgicale, durant une intervention chirurgicale, en postopératoire, et l'une de leurs combinaisons.

10. Composition pour une utilisation selon la revendication 1, où ladite administration minimise ou prévient la formation de cicatrices.

11. Composition pour une utilisation selon la revendication 6, où une cause de ladite plaie dans la chair est choisie dans le groupe constitué d'une incision, une lacération, une brûlure thermique, une brûlure chimique, une abrasion, une plaie par perforation, et l'une de leurs combinaisons.

12. Procédé de prévention ou de réduction de l'apparition d'une ride comprenant l'administration à une région de la peau qui développe généralement une ride ou à une ride existante d'une composition cosmétique comprenant une quantité efficace d'un composé ayant une structure : 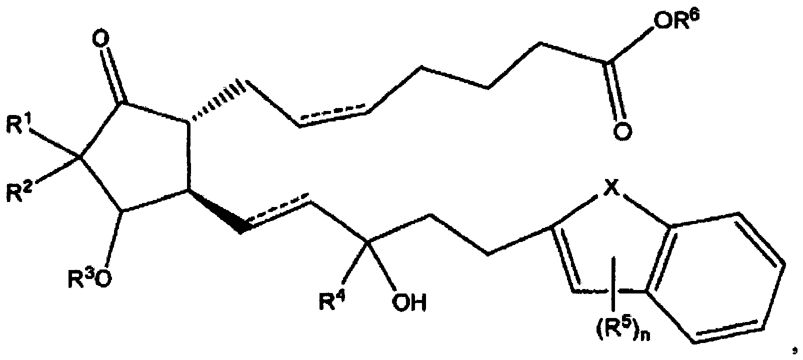 dans laquelle chaque ligne en pointillés représente la présence ou l'absence d'une double liaison ;
R¹, R², R³ et R⁴ sont choisis chacun indépendamment parmi H et un groupe alkyle en C₁ à C₆ linéaire ; R⁵ représente un atome d'halogène, un groupe alkyle en C₁ à C₆, ou alcényle en C₁ à C₆ ; R⁶ représente H, un groupe alkyle en C₁ à C₆, alcényle en C₁ à C₆, l'un de ses sels, ou l'une de ses amines ; n vaut 0 à 7 ; et X représente S ou O.

13. Procédé selon la revendication 12, dans lequel le composé est :

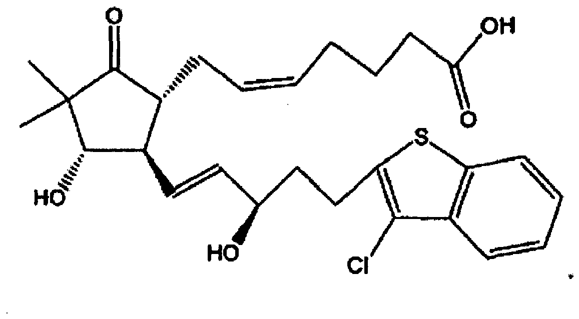

14. Procédé selon la revendication 12, dans lequel ladite composition est administrée par voie topique.

15. Procédé selon la revendication 12, dans lequel la ride est choisie dans le groupe constitué d'une ride due au froncement des sourcils, les pattes d'oie, les plis nasogéniens, une ride sous l'oeil, un creux entre les sourcils, et l'une de leurs combinaisons.
